(19) Europäisches Patentamt / European Patent Office / Office européen des brevets

(11) **EP 3 906 954 B1**

(12) **FASCICULE DE BREVET EUROPEEN**

(45) Date de publication et mention
de la délivrance du brevet:
**04.01.2023 Bulletin 2023/01**

(21) Numéro de dépôt: **21177522.6**

(22) Date de dépôt: **28.10.2020**

(51) Classification Internationale des Brevets (IPC):
*A61M 16/00* ^(2006.01)   *A61M 16/10* ^(2006.01)
*A61M 16/12* ^(2006.01)   *G16H 20/00* ^(2018.01)
*G16H 40/00* ^(2018.01)

(52) Classification Coopérative des Brevets (CPC):
**A61M 16/024; A61M 16/1005; A61M 16/12;
A61M 16/204; G16H 20/40; G16H 40/63;**
A61M 16/0833; A61M 16/085; A61M 16/0891;
A61M 16/1065; A61M 16/16; A61M 16/22;
A61M 2016/0039; A61M 2016/1035;
A61M 2202/0208;                               (Cont.)

(54) **DISPOSITIF DE FOURNITURE DE GAZ THÉRAPEUTIQUE, EN PARTICULIER DE NO OU DE N2O, À UN PATIENT**

VORRICHTUNG ZUR ZUFÜHRUNG VON THERAPEUTISCHEM GAS, INSBESONDERE NO ODER N2O, ZU EINEM PATIENTEN

DEVICE FOR SUPPLYING THERAPEUTIC GAS, IN PARTICULAR NO OR N2O, TO A PATIENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **15.11.2019 FR 1912769**

(43) Date de publication de la demande:
**10.11.2021 Bulletin 2021/45**

(62) Numéro(s) de document de la (des) demande(s)
initiale(s) en application de l'article 76 CBE:
**20204460.8 / 3 821 929**

(73) Titulaires:
• **L'Air Liquide, société anonyme pour l'Étude
et l'Exploitation des procédés Georges Claude
75007 Paris (FR)**
• **Air Liquide Santé France
75007 Paris (FR)**
Etats contractants désignés:
**FR**

(72) Inventeurs:
• **BLANDIN, Yann
94250 Gentilly (FR)**
• **THEVENET, Louise
92450 Gentilly (FR)**
• **AMORY, Marie
44980 Sainte Luce sur Loire (FR)**

(74) Mandataire: **Air Liquide
L'Air Liquide S.A.
Direction de la Propriété Intellectuelle
75, Quai d'Orsay
75321 Paris Cedex 07 (FR)**

(56) Documents cités:
WO-A1-2012/094008    WO-A2-02/40914
JP-A- H11 192 303    US-A1- 2003 116 159
US-A1- 2015 273 175    US-A1- 2015 320 951

(52) Classification Coopérative des Brevets (CPC):
(Cont.)
A61M 2202/0275; A61M 2202/0283;
A61M 2205/3334; A61M 2205/505; A61M 2205/52;
A61M 2205/8206; F17C 2250/032;
F17C 2250/0443; F17C 2250/0473

**Description**

La présente invention concerne un dispositif de fourniture de gaz thérapeutique, en particulier de monoxyde d'azote (NO) ou de protoxyde d'azote ($N_2O$), et par ailleurs une installation d'administration de gaz à un patient comprenant un tel dispositif de fourniture de gaz thérapeutique.

Le monoxyde d'azote inhalé ou NOi est un médicament gazeux couramment utilisé pour traiter des patients souffrant d'hypertension artérielle pulmonaire aiguë, en particulier les vasoconstrictions pulmonaires chez l'adulte ou l'enfant, y compris les nouveau-nés (PPHN), comme décrit par exemple par EP-A-560928 ou EP-A-1516639.

Une installation de mise en œuvre d'un traitement par NOi, couramment appelée installation d'administration de NO, comprend classiquement une ou des bouteilles de mélange $NO/N_2$ alimentant un dispositif d'administration et/ou de monitoring du traitement par NO, un ventilateur médical pour délivrer un gaz respiratoire, tel un mélange $O_2/N_2$ ou de l'air, auquel est ajouté le NO (i.e. $NO/N_2$) et un kit patient comprenant notamment une interface respiratoire, telle une sonde trachéale, et un ou plusieurs conduits flexibles.

Une telle installation d'administration de NO est utilisée en milieu hospitalier pour administrer le traitement par NOi et ainsi soigner les patients de l'hôpital ayant besoin d'inhaler du NO pour traiter leur hypertension artérielle pulmonaire.

Des exemples de telles installations d'administration de NO sont donnés par les documents WO-A-2012/094008, US-A-2015/320951, US-A-2015/273175, JP-AH11192303, WO-A-02/40914 et US-A-2003/116159.

Or, la durée de traitement des patients varie d'un patient à l'autre, à savoir de quelques heures à plusieurs jours. Dès lors, au sein des hôpitaux, il est très difficile de tracer et d'enregistrer le nombre de patients ayant été traités par NOi sur une période donnée, par exemple un ou plusieurs mois, voire une année entière, et ce, contrairement aux autres médicaments, alors que cette information du nombre de patients traités est la plus pertinente pour les hôpitaux qui sont tenus de garantir le bon usage du médicament et de corréler le nombre de prescriptions avec leur activité hospitalière.

Une problématique similaire existe par ailleurs pour d'autres gaz thérapeutiques, en particulier les mélanges équimolaires d'oxygène et protoxyde d'azote (MEOPA), i.e. les mélanges $O_2/N_2O$, voire d'autres gaz médicaux.

Pour tenter de résoudre ce problème, certains dispositifs d'administration ou bouteilles de gaz comptabilisent le nombre d'heures d'utilisation du gaz thérapeutique de manière à en déduire un nombre approximatif de patients traités à partir d'une durée moyenne d'utilisation fixée.

Or, on comprend que cette façon de procéder n'est pas idéale car basée sur une durée moyenne unique, alors que les durées d'utilisation sont très variables d'un patient à un autre. Ainsi, on comprend aisément que les quantités de gaz utilisées chez les adultes sont très différentes de celles utilisées chez un nouveau-né. Le calcul qui en résulte est donc forcément imprécis, voire même totalement erroné.

De plus, cette façon de faire conduit aussi à des erreurs car elle repose sur des calculs faits par l'utilisateur, lequel peut se tromper en réalisant ces calculs.

Enfin, si une bouteille de gaz reste ouverte par inadvertance, le gaz débité sera considéré comme ayant été utilisé pour soigner des patients, alors qu'il a été perdu, ce qui faussera là encore le calcul.

Le problème est dès lors de pouvoir opérer un comptage précis et fiable du nombre de patients traités par inhalation de gaz thérapeutique, notamment de NO, qui soit consultable à tout moment par l'utilisateur, tel le personnel soignant, et qui soit automatisé afin de fournir une information utile à l'utilisateur et ce, de manière instantanée, en particulier sans qu'il ait à opérer lui-même le moindre calcul, en particulier dans le cadre de la fourniture de mélanges $NO/N_2$ à un patient en ayant besoin, notamment pour traiter leur hypertension artérielle pulmonaire ou une hypo-oxygénation de leur sang.

Une solution selon l'invention concerne alors un dispositif de fourniture de gaz thérapeutique comprenant :

- un passage interne pour acheminer un flux de gaz thérapeutique entre (au moins) une entrée et (au moins) une sortie de gaz,
- des moyens à valve pour contrôler le flux de gaz thérapeutique dans le passage interne,
- des moyens de pilotage comprenant au moins un microprocesseur configurés pour contrôler au moins les moyens à valve,
- un afficheur graphique configuré pour afficher (au moins) des choix sélectionnables par un utilisateur, et éventuellement d'autres informations, et
- des moyens de sélection comprenant une ou des touches de sélection, configurés pour opérer une sélection parmi les choix sélectionnables affichés sur l'afficheur graphique,

caractérisé en ce que les moyens de pilotage sont configurés pour :

i) comptabiliser un nombre total (N) de patients traités par administration du gaz thérapeutique à partir de la sélection par un utilisateur, via les moyens de sélection, d'un premier choix donné correspondant au début d'un traitement par administration du gaz thérapeutique à un patient considéré, ladite comptabilisation du nombre total (N) de patients traités étant opérée par incrémentation d'un compteur de patients,

ii) déterminer une durée totale de traitement pour chaque patient traité correspond à la période de temps s'écoulant entre une sélection par l'utilisateur :

- du premier choix donné correspondant au début d'un traitement par administration du gaz thérapeutique à un patient considéré et
- d'un second choix donné correspondant à la fin du traitement par administration du gaz thérapeutique audit patient considéré,

iii) déterminer, à partir du nombre total (N) de patients traités ayant été comptabilisé et des durées totales de traitement déterminées pour les patients traités :

- le nombre de traitements courts (TC) pour lesquels la durée totale de traitement est inférieure à une durée-seuil préfixée et/ou
- le nombre de traitements longs (TL) pour lesquels la durée totale de traitement est supérieure à ladite durée-seuil préfixée, et

iv) coopérer avec l'afficheur graphique pour afficher le nombre total (N) de patients traités ayant été comptabilisé, le nombre de traitements courts (TC) et/ou le nombre de traitements longs (TL),

et dans lequel :

- l'afficheur graphique comprend un écran tactile et la ou les touches de sélection sont une ou des touches virtuelles s'affichant sur l'écran tactile, et
- les moyens de pilotage sont configurés pour commander les moyens à valve et débuter une fourniture de gaz thérapeutique en réponse à une sélection par l'utilisateur du premier choix donné, par appui sur une touche virtuelle s'affichant sur l'écran tactile, correspondant au début d'un traitement par administration de gaz thérapeutique au patient considéré, et pour incrémenter de +1 le compteur du nombre total (N) de patients traités, après ledit appui sur la touche virtuelle.

Selon le mode de réalisation considéré, le dispositif de fourniture de gaz thérapeutique de l'invention peut comprendre l'une ou plusieurs des caractéristiques suivantes :

- les moyens de pilotage comprennent au moins un microcontrôleur.
- ledit au moins un microprocesseur est agencé sur une carte électronique.
- les moyens de pilotage comprennent au moins un microprocesseur mettant en œuvre un ou plusieurs algorithmes.
- les moyens de sélection comprennent des touches de sélection.
- l'afficheur graphique comprend un écran numérique (i.e. digital).
- la ou les touches de sélection sont une ou des touches tactiles.
- l'afficheur graphique comprend un écran d'affichage en couleurs ou en noir et blanc, ou les deux.
- les moyens de pilotage sont configurés pour comptabiliser un nombre total (N) de patients traités par incrémentation d'un compteur interne du microprocesseur.
- les moyens de pilotage sont configurés pour incrémenter de +1, le nombre total (N) de patients traités, en réponse à une sélection par l'utilisateur du premier choix donné correspondant au début d'un traitement par administration de gaz thérapeutique à un patient considéré.
- les moyens de pilotage sont configurés pour commander les moyens à valve et débuter une fourniture de gaz thérapeutique en réponse à une sélection par l'utilisateur du premier choix donné correspondant au début d'un traitement par administration de gaz thérapeutique à un patient considéré suivie d'une validation (i.e. confirmation) de ladite sélection du premier choix donné, de préférence ladite validation est opérée par sélection par l'utilisateur d'une touche de validation, en particulier une touche de validation virtuelle affichant sur l'afficheur graphique.
- les moyens de pilotage sont en outre configurés pour :

    ▪ interrompre temporairement la détermination de la durée totale de traitement pour un patient considéré après :

    - soit sélection par l'utilisateur du second choix correspondant à la fin du traitement,
    - soit sélection par l'utilisateur d'un troisième choix donné correspondant à une suspension de traitement avec interruption temporaire de l'administration de gaz thérapeutique audit patient considéré, et

■ reprendre la détermination de la durée totale de traitement pour ledit patient considéré après :

- soit re-sélection par l'utilisateur du premier choix donné correspondant au début d'un traitement par administration du gaz thérapeutique, c'est-à-dire une sélection supplémentaire du premier choix chez un même patient,
- soit sélection par l'utilisateur d'un quatrième choix donné correspondant à la reprise d'un traitement par administration du gaz thérapeutique chez ledit patient donné.

- les moyens de pilotage sont en outre configurés pour ne pas incrémenter le compteur de patients après une re-sélection par l'utilisateur du premier choix correspondant au début d'un traitement ou après une sélection du quatrième choix correspondant à une reprise de traitement chez un patient donné. En effet, une re-sélection du premier choix ou une sélection du quatrième choix est interprétée/considérée par les moyens de pilotage, en particulier le microprocesseur, comme une reprise d'un même traitement chez un même patient, suite à une suspension momentanée de ce traitement chez ce patient.
- les touches de sélection comprennent :

o une touche « Début de traitement » commandant un début de fourniture de gaz thérapeutique et
o une touche « Fin de traitement » commandant un arrêt de fourniture de gaz thérapeutique.

- la touche « Début de traitement » et la touche « Fin de traitement » sont une seule et même touche commandant le début et la fin de la fourniture de gaz.
- la touche « Début de traitement » et la touche « Fin de traitement » sont des touches différentes.
- les touches de sélection comprennent en outre éventuellement :

■ une touche « Suspension de traitement » commandant une suspension de la fourniture de gaz thérapeutique et/ou
■ une touche « Reprise de traitement » commandant une reprise (après suspension) de la fourniture de gaz thérapeutique.

- la touche « Suspension de traitement » et la touche « Reprise de traitement » sont une seule et même touche commandant la suspension et la reprise de la fourniture de gaz.
- la touche « Suspension de traitement » et la touche « Reprise de traitement » sont des touches différentes.
- selon un autre mode de réalisation, la touche « Suspension de traitement » et la touche « Fin de traitement » sont une seule et même touche commandant un arrêt définitif ou temporaire (i.e. suspension) de la fourniture de gaz.
- selon encore un autre mode de réalisation, la touche « Début de traitement » et la touche « Reprise de traitement » sont une seule et même touche commandant un début (i.e. initial ou reprise après suspension) de fourniture de gaz.
- le nombre TC correspond au nombre de patients ayant été soumis à un traitement ayant été interrompu rapidement, typiquement après moins de 3 heures, car ce traitement n'était pas efficace, engendrait des effets secondaires négatifs ou pour une autre raison.
- le nombre TL correspond au nombre de patients ayant été soumis à un traitement de longue durée car efficace chez ces patients, par exemple sur une durée pouvant atteindre plusieurs jours.
- selon un mode de réalisation particulier, les moyens de pilotage sont en outre configurés pour ne pas incrémenter de +1, le nombre total (N) de patients traités (i.e. ne pas comptabiliser) lorsque la durée totale de traitement déterminée est inférieure à une durée minimale préfixée qui est inférieure à la durée-seuil préfixée, par exemple une durée minimale préfixée inférieure ou égale à 1 heure, de préférence inférieure ou égale à 30 minutes. En effet, utiliser le dispositif durant une durée minimale courte correspond en général à une situation exceptionnelle ne correspondant pas à un traitement de patient, par exemple à la correction d'une erreur de réglage, à une démonstration lors d'une formation ou autre, laquelle situation exceptionnelle ne doit pas être comptabilisée pour ne pas fausser le calcul du nombre total (N) de patients traités.
- le nombre de traitements longs (TL) est égal au nombre total (N) de patients traités duquel est déduit le nombre de traitements courts (TC), c'est-à-dire :

$$TL = N - TC. \text{ Dit autrement, } N = TC + TL \text{ ou } TC = N - TL.$$

- les moyens de pilotage coopèrent avec l'afficheur graphique pour afficher en outre le nombre de traitements courts (TC ) et/ou le nombre de traitements longs (TL).

- il comprend en outre des moyens de mémorisation configurés pour mémoriser :

  - le nombre total (N) de patients traités,
  - le nombre de traitements courts (TC),
  - le nombre de traitements longs (TL),
  - les choix sélectionnables par l'utilisateur et/ou
  - la durée-seuil préfixée,
  - et/ou éventuellement la durée minimale préfixée.

- la durée-seuil est inférieure ou égale à 5 heures, typiquement de l'ordre de 3h à 4h, par exemple de 3h, lorsque le gaz thérapeutique est du NO.
- la durée minimale est inférieure ou égale à 1 h, par exemple de l'ordre de 30 minutes.
- la durée-seuil est ajustable et/ou la durée minimale est ajustable.
- les moyens de mémorisation comprennent une ou plusieurs mémoires d'enregistrement et de stockage de données, par exemple une ou des mémoires volatiles ou non-volatiles, par exemple de type mémoire flash ou autre.
- au moins une mémoire (e.g. flash) est agencée sur une ou la carte électronique.
- il comprend en outre des moyens d'alimentation en courant électrique servant à alimenter en courant électrique les composants du dispositif qui en requièrent pour fonctionner, tel que l'afficheur, le microprocesseur, la mémoire...
  Les moyens d'alimentation en courant électrique peuvent comprendre, de manière classique, une (ou des) batterie rechargeable ou non, un cordon électrique et une prise d'alimentation connectable au secteur électrique (110/220 V), un transformateur de courant...
- il comprend en outre une carcasse ou un boitier rigide externe dans lequel sont agencés tout ou partie des composants du dispositif, en particulier les moyens de pilotage, le passage interne, les moyens à valve...
- l'afficheur graphique est agencé dans une paroi de la carcasse rigide externe.
- les moyens de pilotage sont configurés pour contrôler (au moins) les moyens à valve de manière à ajuster le débit de gaz thérapeutique circulant dans le passage interne.
- les moyens à valve comprennent au moins une (électro)vanne proportionnelle ou plusieurs électrovannes, de préférence plusieurs électrovannes agencées en parallèle.
- il comprend en outre un dispositif débitmètre agencé sur le passage de gaz pour déterminer (i.e. mesurer) le débit de gaz thérapeutique circulant dans le passage interne.
- le dispositif débitmètre est du type à fil chaud ou à perte de charge.
- le dispositif débitmètre coopère avec les moyens de pilotage.
- il comprend des moyens de remise à zéro (RAZ) permettant de réinitialiser le ou les compteurs, par exemple une touche de RAZ ou analogue .

L'invention concerne aussi une installation d'administration de gaz à un patient comprenant :

- au moins une source de gaz thérapeutique,
- un dispositif de fourniture de gaz thérapeutique selon l'invention, alimenté en gaz thérapeutique par ladite au moins une source de gaz thérapeutique, et
- une ligne d'alimentation en gaz alimenté en gaz thérapeutique par le dispositif de fourniture de gaz thérapeutique.

Selon le mode de réalisation considéré, l'installation d'administration de gaz de l'invention peut comprendre l'une ou plusieurs des caractéristiques suivantes :

- elle comprend en outre un ventilateur médical, c'est-à-dire un appareil d'assistance ventilatoire, en communication fluidique avec la ligne d'alimentation en gaz.
- le ventilateur médical est relié fluidiquement à la ligne d'alimentation pour alimenter ladite ligne d'alimentation avec de l'air ou un mélange $N_2/O_2$.
- la (ou les) source de gaz thérapeutique contient du NO, en particulier un mélange $NO/N_2$.
- la source de gaz thérapeutique contient un mélange $NO/N_2$ contenant moins de 2000 ppm en volume de NO, le reste étant de l'azote, de préférence moins de 1000 ppm en volume de NO, le reste étant de l'azote
- préférentiellement, la source de gaz thérapeutique contient un mélange $NO/N_2$ contenant de 250 à 900 ppm en volume de NO, le reste étant de l'azote, par exemple de l'ordre de 800 ppm en volume de NO, le reste étant de l'azote.
- alternativement, au moins une source de gaz thérapeutique contient un mélange $O_2/N_2O$, de préférence un mélange équimolaire (50%/50% mol) d'oxygène et de protoxyde d'azote, c'est-à-dire du MEOPA.
- alternativement, la source de gaz thérapeutique contient de l'oxygène, en particulier elle comprend un récipient d'oxygène ou une canalisation d'oxygène.

- alternativement, la source de gaz thérapeutique contient de l'argon ou un mélange argon/oxygène.
- elle comprend en outre un humidificateur de gaz agencé sur la ligne d'alimentation en gaz, de préférence en aval du site où le dispositif de fourniture de gaz thérapeutique est raccordé fluidiquement à ladite ligne d'alimentation en gaz de manière à l'alimenter en gaz thérapeutique.
- elle comprend en outre une ligne de récupération des gaz expirés par le patient.
- la ligne d'alimentation en gaz et la ligne de récupération des gaz expirés sont raccordées à une pièce de raccordement, de préférence une pièce en Y, et définissent un circuit patient.
- la ligne d'alimentation en gaz forme une branche inspiratoire du circuit patient.
- la ligne de récupération des gaz expirés forme une branche expiratoire du circuit patient.
- la ligne d'alimentation en gaz est raccordée fluidiquement à un port de sortie du ventilateur médical de manière à récupérer et acheminer le gaz délivré par le ventilateur médical.
- la ligne de récupération des gaz expirés est raccordée fluidiquement à un port d'entrée du ventilateur médical de manière à fournir au ventilateur médical tout ou partie des gaz expirés par le patient et à déterminer ainsi une ou des éventuelles fuites de gaz.
- la ligne de récupération des gaz expirés comprend éventuellement (i.e. non obligatoire) un dispositif d'élimination du $CO_2$ permettant d'éliminer le $CO_2$ présent dans les gaz expirés par le patient.
- la ligne de récupération des gaz expirés comprend éventuellement (i.e. non obligatoire) un filtre.
- au moins une source de gaz thérapeutique comprend un ou plusieurs récipients de gaz, en particulier une ou des bouteilles de gaz sous pression.
- le (ou les) récipient de gaz est équipé d'un robinet de distribution de gaz.
- le robinet de distribution de gaz est un robinet avec ou sans détenteur intégré (RDI).
- le robinet de distribution de gaz est en alliage de cuivre, tel du laiton.
- le (ou les) récipient de gaz est équipé d'un capotage de protection agencé autour du robinet de distribution de gaz.
- le capotage est en matériau polymère (i.e. plastique), en métal ou leurs combinaisons.
- le (ou les) récipient de fluide est une bouteille de gaz sous pression contenant, lorsqu'il est plein, un mélange gazeux, en particulier $NO/N_2$, à une pression d'au moins 150 à 200 bar abs, voire d'au moins 250 à 300 bar abs.
- le récipient de fluide a une forme générale cylindrique, en particulier d'ogive.

Il est également décrit une méthode de traitement thérapeutique comprenant une administration d'un gaz thérapeutique à un patient, en particulier du NO, tel un mélange NO/azote, dans laquelle on utilise une installation d'administration de gaz et/ou un dispositif de fourniture de gaz thérapeutique selon l'invention afin de fournir ledit gaz thérapeutique audit patient, en particulier du NO, tel un mélange NO/azote, à un patient souffrant d'hypertension pulmonaire, que ce soit un adulte, un adolescent, un enfant ou un nouveau-né, par exemple un nouveau-né souffrant d'hypertension pulmonaire persistante (PPHN).

Selon le mode de réalisation considéré, le mélange NO/azote peut être dilué avec de l'air ou un mélange azote/oxygène avant d'être fourni au patient. De préférence, l'air ou le mélange azote/oxygène est fourni par un ventilateur médical.

L'invention va maintenant être mieux comprise grâce à la description détaillée suivante, faite à titre illustratif mais non limitatif, en référence aux figures annexées parmi lesquelles :

Fig. 1 schématise un mode de réalisation d'un dispositif de fourniture de gaz thérapeutique selon l'invention,
Fig. 2 schématise un mode de réalisation d'une installation d'administration de gaz selon l'invention, et
Fig. 3 schématise un mode de réalisation d'un écran du dispositif de fourniture de gaz selon l'invention.

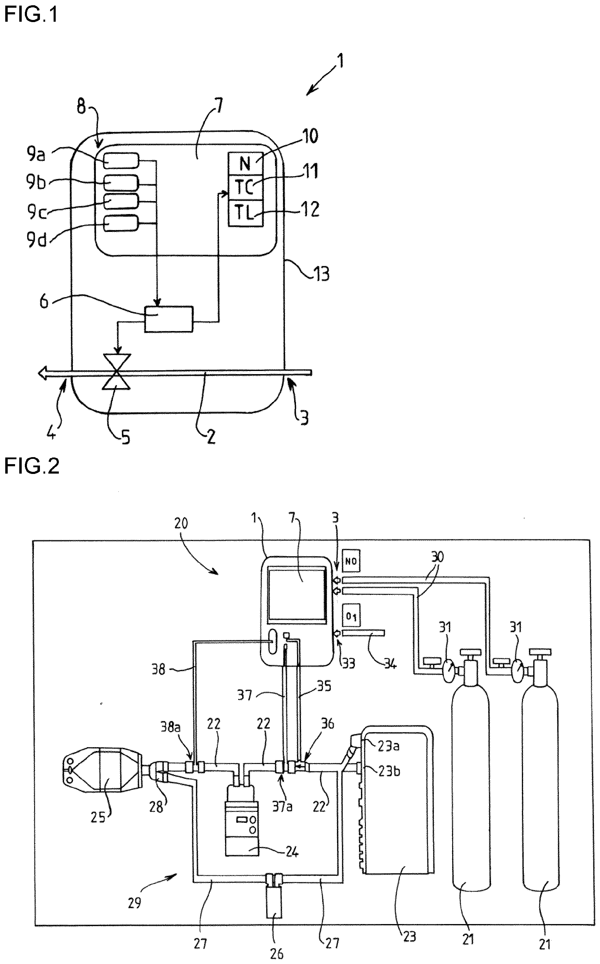

Fig. 1 schématise un mode de réalisation d'un dispositif de fourniture de gaz thérapeutique 1 selon l'invention comprenant un boitier 13 rigide, par exemple en polymère, au sein duquel est agencé un passage de gaz interne 2, tel un conduit de gaz ou analogue, pour acheminer un flux de gaz thérapeutique.

Dans ce mode de réalisation, on prend comme exemple de gaz thérapeutique, un flux gazeux à base de NO, c'est-à-dire un mélange de monoxyde d'azote et d'azote ou $NO/N_2$.

Le flux gazeux à base de NO entre et circule dans le passage de gaz interne 2 entre une (ou plusieurs) entrée 3 et une sortie 4 de gaz. Les entrée 3 et sortie 4 de gaz peuvent par exemple être portées par des connecteurs ou embouts de raccordement mécanique et fluidique portés par le boitier 13 du dispositif 1, auxquels viennent se fixer des lignes de gaz servant à convoyer du gaz, comme expliqué ci-après, par exemples des tuyaux flexibles ou analogues.

Des moyens à valve 5, i.e. un (ou des) dispositif à valve, par exemple une pluralité d'électrovannes agencées en parallèle, sont agencés sur le passage de gaz interne 2 et permettent de contrôler le flux de gaz thérapeutique qui circule dans le passage interne 2, dans le sens allant de l'entrée 3 à la sortie 4 de gaz. Bien entendu, on peut aussi utiliser d'autres moyens à valve, par exemple une ou des (électro)vannes proportionnelles.

Les moyens à valve 5 sont commandés par des moyens de pilotage 6, i.e. un (ou des) dispositif de pilotage, agencés dans le boitier 13, typiquement une carte électronique comprenant un (ou plusieurs) microprocesseur(s), typiquement un (ou des) microcontrôleur, mettant en œuvre un ou des algorithmes. Les moyens de pilotage 6 permettent notamment d'ajuster ou contrôler le débit de gaz traversant les moyens à valve 5.

Ainsi, les moyens de pilotage 6 peuvent piloter les moyens à valve 5, telles des électrovannes agencées en parallèle, c'est-à-dire ouvrir ou fermer toutes ou partie de ces vannes, pour obtenir un débit de gaz (Q) déterminé/calculé par le microcontrôleur à partir d'une valeur réglée/fixée par exemple par l'utilisateur et en fonction du débit (Q') de gaz, i.e. air, délivré par le ventilateur 23, comme expliqué ci-après.

On prévoit aussi un (ou des) débitmètre (non représenté) agencé sur le passage de gaz interne 2, en amont et/ou en aval des moyens à valve 5 pour déterminer le débit de gaz (Q) à base de NO. Le débitmètre peut être du type à différentiel de pression, à fil chaud ou autre. Il coopère avec les moyens de pilotage 6 pour leur fournir des mesures.

Par ailleurs, le boitier 13 comprend un afficheur graphique 7 comprenant un écran tactile, servant à afficher différentes informations ou données, en particulier différents choix sélectionnables 9a-9d par un utilisateur. Ces choix peuvent s'afficher dans des fenêtres ou analogues s'affichant sur l'écran tactile/digital de l'afficheur graphique 7.

L'afficheur graphique 7 est aussi configuré pour visualiser une ou des courbes, graphiques, alarmes, icones... ou toute autre information ou donnée utile à l'utilisateur, tel le personnel soignant.

Il est également prévu des moyens de sélection 8, i.e. un (ou des) dispositif de sélection, permettant à l'utilisateur d'opérer une sélection parmi les choix sélectionnables 9a-9d qui sont affichés sur afficheur graphique 7, c'est-à-dire de choisir entre plusieurs options proposées et s'affichant à l'écran, ou de confirmer/valider ou de refuser un choix (i.e. une option), ou encore d'opérer des réglages ou ajustage, par exemple de sélectionner la valeur de débit souhaitée.

Les moyens de sélection 8 comprennent typiquement des touches ou boutons de sélection actionnables par l'utilisateur. Avantageusement, les moyens de sélection 8 sont des touches virtuelles à actionnement tactile, c'est-à-dire des touches tactiles, s'affichant sur l'afficheur graphique 7 qui est lui-même un écran tactile.

Selon la présente invention, les moyens de pilotage 6 sont aussi configurés pour comptabiliser un nombre total (N) de patients traités par administration du gaz thérapeutique à partir de la sélection par un utilisateur, via les moyens de sélection 8, en particulier une touche tactile affichée sur l'afficheur graphique 7, d'un premier choix donné 9a correspondant au début d'un traitement par administration du gaz thérapeutique à un patient considéré. Par exemple, le début d'un traitement peut être lancé par action digitale de l'utilisateur sur une touche « Début de traitement » commandant le début de fourniture de gaz thérapeutique.

La comptabilisation du nombre total (N) de patients traités se fait par incrément de +1 d'un compteur, par exemple un compteur du microprocesseur ou autre.

Dit autrement, dès qu'un traitement par administration de gaz thérapeutique, tel du NO, doit débuter chez un patient, l'utilisateur appuie sur la touche tactile « Début de traitement » affichée sur l'afficheur graphique 7 correspond au lancement, i.e. début, de ce traitement, ce qui incrémente de +1, le compteur qui comptabilise le nombre total (N) de patients traités.

Le nombre total (N) de patients traités peut être affiché sur l'afficheur graphique 7, soit en permanence, soit à la demande de l'utilisateur, par exemple après appui sur une touche dédiée.

Comme illustré en Fig. 1, l'afficheur graphique 7 peut afficher plusieurs choix sélectionnables 9a-9d correspondant chacun à une action donnée spécifique.

Par exemple, l'afficheur 7 peut afficher sous forme de touches ou analogues :

- un premier choix donné 9a correspondant à un début du traitement par administration du gaz thérapeutique au patient, c'est-à-dire une touche « Début de traitement » ;
- un second choix donné 9b correspondant à la fin du traitement chez le patient considéré, c'est-à-dire une touche « Fin de traitement » ;
- un troisième choix donné 9c correspondant à une suspension du traitement avec interruption temporaire de l'administration de gaz thérapeutique chez le patient considéré, c'est-à-dire à une mise en pause du traitement par sélection d'une touche « Suspension de traitement » ; et
- un quatrième choix donné 9d correspondant à la reprise du traitement chez le patient donné par sélection d'une touche « Reprise de traitement ».

La sélection d'un de ces choix 9a-9d par exemple par appui digital de l'utilisateur sur la touche virtuelle, par exemple une fenêtre ou analogue, de l'écran tactile de l'afficheur 7, va être transmise au microprocesseur des moyens de pilotage 6 et celui-ci va piloter le fonctionnement de l'appareil en fonction de la sélection opérée.

Par exemple, les moyens de pilotage 6 peuvent commander les moyens à vannes 5 pour :

- autoriser une circulation de gaz dans le passage interne 2, au travers de la ou des électrovannes de contrôle de flux en fonction du débit (Q) fixé, en direction du patient en cas d'appui sur la touche correspondant au premier choix 9a, c'est-à-dire correspondant à un début de traitement par administration du gaz thérapeutique au patient, c'est-à-dire la touche « Début de traitement » ;

- interrompre la circulation de gaz dans le passage interne 2 en direction du patient en cas d'appui sur la touche correspondant au deuxième ou troisième choix 9b ou 9c, donc à un arrêt définitif ou temporaire du traitement chez le patient, c'est-à-dire une touche de « Fin de traitement » ou de « Suspension de traitement » ; et
- autoriser à nouveau circulation de gaz dans le passage interne 2 en direction du patient en cas d'appui sur la touche correspondant au quatrième choix 9d, subséquemment à un appui sur la touche de troisième choix 9c synonyme d'arrêt temporaire du traitement, i.e. de suspension momentanée du traitement, par sélection de la touche « Reprise de traitement ».

Dans le mode de réalisation de Fig. 1, les 4 touches 9a-9d sont individuelles et commandent chacune une action spécifique. Toutefois, certaines des actions/choix pourraient, selon d'autres modes de réalisation, être sélectionnés par une même touche, c'est-à-dire qu'une même touche pourrait servir à sélectionner plusieurs choix différents.

Par exemple, les troisième et quatrième choix 9c, 9d sont affichés sous forme de deux touches virtuelles ou analogues de « Suspension de traitement » et de « Reprise de traitement ». Toutefois, selon un autre mode de réalisation, ces troisième et quatrième choix 9c, 9d pourraient être sélectionnés par une seule et même touche virtuelle de « Suspension/Reprise de traitement ».

De même, selon encore un autre mode de réalisation, les premier, troisième et quatrième choix 9a, 9c, 9d pourraient être sélectionnés par une seule et même touche virtuelle de « démarrage/suspension/reprise » de traitement.

Selon encore un autre mode de réalisation, une même touche pourrait commander le début et la reprise du traitement après suspension et une autre touche pourrait commander la suspension temporaire et la fin définitive du traitement.

Bien entendu, d'autres combinaisons ou modes de réalisation sont aussi possibles.

Dans tous les cas, un appui, i.e. sélection par l'utilisateur, sur la touche ou analogue correspondant au premier choix 9a, c'est-à-dire à un début de traitement chez un patient considéré (i.e. nouveau patient), engendre une incrémentation de +1 du compteur du nombre de patients traités et une nouvelle incrémentation de +1 ne peut avoir lieu qu'après appui sur la touche correspondant au second choix 9b, c'est-à-dire la touche signalant une fin définitive de traitement du patient considéré. Il est à souligner qu'une sélection des choix 9c, 9d correspondant à une suspension et/ou à une reprise de traitement n'engendre aucune incrémentation du compteur.

Selon une variante de réalisation, le début d'un traitement par administration de gaz thérapeutique à un patient ne peut débuter qu'après une validation, i.e. confirmation, par l'utilisateur du premier choix sélectionné par appui sur la touche de « Début de traitement », de préférence la validation est opérée par sélection par l'utilisateur d'une touche de validation dédiée, en particulier une touche de validation virtuelle affichant sur l'afficheur graphique 7, comme détaillé ci-dessous.

Autrement dit, les moyens de pilotage 6 sont configurés pour commander les moyens à valve 5 et débuter une fourniture de gaz thérapeutique uniquement après sélection par l'utilisateur du premier choix donné de « Début de traitement » puis de la confirmation de ce choix par appui sur la touche de validation.

Les moyens de pilotage 6 sont aussi configurés pour déterminer une durée totale de traitement pour chaque patient traité correspond à la période de temps s'écoulant entre une sélection par l'utilisateur du premier choix donné 9a correspondant au début d'un traitement par administration du gaz thérapeutique à un patient considéré et du second choix donné 9b correspondant à la fin définitive du traitement par administration de gaz thérapeutique au patient considéré.

De ce fait, les moyens de pilotage 6 sont configurés pour interrompre, i.e. stopper, temporairement la détermination de la durée totale de traitement pour un patient considéré après sélection par l'utilisateur du troisième choix donné 9c correspondant à une suspension de traitement avec interruption temporaire de l'administration de gaz thérapeutique au patient considéré, et ensuite reprendre la détermination de la durée totale de traitement après :

- soit re-sélection par l'utilisateur du premier choix donné 9a correspondant au début d'un traitement par administration du gaz thérapeutique,
- soit sélection d'un quatrième choix donné 9d correspondant à la reprise d'un traitement par administration du gaz thérapeutique chez le patient considéré.

Les moyens de pilotage 6 sont aussi configurés pour déterminer :

- le nombre de traitements courts (TC) pour lesquels la durée totale de traitement est inférieure à une durée-seuil préfixée, i.e. le nombre de patients ayant été soumis à un tel traitement de courte durée, par exemple moins de 3 h, et/ou
- le nombre de traitements longs (TL) pour lesquels la durée totale de traitement est supérieure à la durée-seuil préfixée, avec TL = N - TC où N est le nombre total de patients traités, i.e. le nombre de patients ayant été soumis à un tel traitement de longue durée, par exemple plus de 3h et pouvant atteindre 1 à plusieurs jours.

Les moyens de pilotage 6 coopèrent avec l'afficheur graphique 7 pour afficher 11, 12 la ou les valeurs ainsi déterminées, à savoir le nombre de traitements courts (TC ) et/ou le nombre de traitements longs (TL).

En effet, pouvoir déterminer et afficher le nombre de traitements courts (TC) et le nombre de traitements longs (TL) parmi le nombre total de patients traités, c'est-à-dire le nombre total de traitements réalisés à l'aide du dispositif considéré, permet de distinguer les patients ayant été réactifs au traitement, c'est-à-dire ayant été soignés par le gaz, de ceux chez qui le gaz n'a pas eu d'effet thérapeutique malgré une administration pendant une durée minimale, c'est-à-dire pouvant atteindre la durée-seuil préfixée. Une telle information est très utile au personnel soignant, notamment pour décider d'un changement éventuel de traitement.

Par exemple, pour un traitement par iNO, ladite durée-seuil peut être fixée à 3 heures ou à une autre durée « courte ». Les traitements stoppés avant 3h seront comptabilisés comme des traitements courts (TC) n'ayant pas eu d'effet thérapeutique notable ou avéré chez les patients, et ceux ayant duré au moins 3h, comme des traitements longs (TL) ayant traités les patients.

De façon générale, le comptage des patients traités avec le dispositif d'administration 1 de gaz de l'invention est géré par un algorithme mis en œuvre par un microprocesseur des moyens de pilotage 6 du dispositif 1. Cet algorithme comptabilise le nombre total (N) de patients traités en incrémentant un compteur. Ce nombre N est affiché à l'écran 7 pour donner cette information à l'utilisateur. Il est préférentiellement enregistré dans une mémoire non-volatile.

Avantageusement, le dispositif 1 permet aussi de prendre en compte le démarrage d'un nouveau traitement complet (i.e. nouveau traitement pour un nouveau patient), une suspension momentanée de traitement, la reprise d'un traitement sur un patient instable en phase de sevrage (i.e. reprise d'un traitement ayant été suspendu) et un traitement 'court' quand la durée d'administration d'un nouveau traitement est inférieure à quelques heures (i.e. durée seuil préfixée), signifiant que le patient ne répond pas au traitement mis en œuvre.

L'événement déclencheur unique engendrant une incrémentation +1 du compteur du nombre total (N) de patients traités est l'action de sélectionner un premier choix 9a s'affichant à l'écran tactile de l'afficheur 7 correspond à "Démarrer un nouveau traitement" par appui d'une touche virtuelle ou analogue s'affichant à l'écran, correspondant à ce premier choix 9a.

Lorsqu'il est nécessaire d'interrompre momentanément le traitement chez un patient donné, le dispositif 1 peut proposer un choix ou une sélection correspondant à une interruption temporaire du traitement sous forme d'une touche dédiée 9c du type « Suspendre le traitement », comme expliqué ci-avant.

Lors de la reprise d'un traitement, par exemple après une interruption de longue durée, le dispositif 1 peut demander une confirmation, i.e. une validation de choix, avant d'incrémenter le compteur, par exemple en demandant à l'utilisateur de sélection une réponse « Oui » ou « Non » à une question du type : "Est-ce un nouveau patient ?" et en proposant deux choix de réponse possible « Oui » et « Non » sous formes de deux touches virtuelles s'affichant à l'écran. Une réponse « non » est traitée par le dispositif 1 comme un redémarrage après une suspension de traitement et non comme un nouveau patient ; le compteur n'est alors pas incrémenté.

Le temps total de traitement est déterminé par les moyens de pilotage 6 comme étant la durée s'étendant entre un appui de l'utilisateur sur la touche 9a signifiant "Démarrer un nouveau traitement" et un appui subséquent sur une autre touche correspondant à un choix 9b du type « arrêter le traitement », voire sur une autre touche correspondant à un troisième choix 9c du type "suspendre le traitement" et non reprise ultérieure du traitement avec le même patient, comme expliqué ci-avant.

Avantageusement, l'afficheur 7 affiche non seulement le nombre total (N) de patients traités (en 10) mais aussi le nombre de traitements courts TC (en 11) et le nombre de traitements longs TL (en 12), c'est-à-dire complets. Le nombre total (N) de patients traités est égal à l'addition du nombre total de traitements longs et du nombre de traitements courts (i.e. N = TL + TC).

L'affichage 10, 11, 12 de tout ou partie des valeurs N, TL et TC peut être disponible en permanence sur l'afficheur graphique 7 ou alors n'apparaître à l'écran qu'après être appelé par l'utilisateur, par exemple par appui sur une touche dédiée ou analogue.

Fig. 2 schématise un mode de réalisation d'une installation d'administration de gaz 20 selon l'invention, comprenant ici deux bouteilles 21 de gaz thérapeutique, à savoir ici des mélanges NO/N$_2$, par exemple contenant 800 ppm vol. de NO (reste N$_2$), qui alimentent en mélange NO/N$_2$, un dispositif de fourniture de gaz thérapeutique 1 selon l'invention, tel qu'illustré en Fig. 1 et décrit ci-avant.

Les bouteilles de gaz 21 sont reliées fluidiquement au dispositif de fourniture de gaz 1 via des lignes d'amenée de gaz 30, tel que des tuyaux flexibles ou analogues, qui peuvent être équipées de dispositifs de régulation et/ou de suivi de la pression de gaz 31, tels que détendeur de gaz, manomètres... Les lignes d'amenée de gaz 30 sont reliées à une ou plusieurs entrées de gaz 3 du dispositif de fourniture de gaz 1 qui alimentent le passage interne 2 dudit dispositif de fourniture de gaz.

On note que le dispositif de fourniture de gaz 1 comprend aussi une entrée d'oxygène 32 reliée fluidiquement, via une ligne d'amenée d'oxygène 34, tel un tuyau flexible ou analogue, à une source d'oxygène par exemple le réseau hospitalier, c'est-à-dire une canalisation d'alimentation en oxygène agencée dans le bâtiment hospitalier.

Par ailleurs, est prévu aussi un ventilateur médical 23, c'est-à-dire un appareil d'assistance respiratoire, pour fournir de l'air ou un mélange oxygène/azote ($N_2/O_2$), c'est-à-dire un flux de gaz respiratoire contenant au moins 21% d'oxygène.

Le ventilateur médical 23 et le dispositif de fourniture de gaz 1 sont en communication fluidique avec une ligne d'alimentation en gaz 22 servant à acheminer le flux gazeux vers le patient.

Le dispositif de fourniture de gaz 1 délivre un mélange $NO/N_2$, par exemple 800 ppmv de NO, dans la ligne d'alimentation en gaz 22, via un conduit d'injection 37, de manière à injecter (en 37a) un flux de $NO/N_2$ dans le flux d'air ou de mélange oxygène/azote délivré par le ventilateur médical 23.

La ligne d'alimentation en gaz 22 comprend en outre un humidificateur de gaz 24 agencé en aval du site (en 36) où le dispositif de fourniture 1 de gaz thérapeutique est raccordé fluidiquement à la ligne d'alimentation 22. Cet humidificateur de gaz 24 permet d'humidifier le flux de gaz, e.g. mélange $NO/N_2/$air, avant qu'il ne soit inhalé par le patient, au moyen d'une interface respiratoire patient, telle une sonde trachéale ou analogue. Sur la Fig. 2, l'ensemble patient/interface est schématisé par un « poumon artificiel » 25.

Est prévue aussi une ligne de récupération 27 des gaz expirés par le patient. La ligne d'alimentation en gaz 22 et la ligne de récupération 27 des gaz expirés sont raccordées à une pièce de raccordement 28, de préférence une pièce en Y, et définissent ainsi un circuit patient 29. La ligne d'alimentation en gaz 22 forme la branche inspiratoire du circuit patient 29, alors que la ligne de récupération 27 des gaz expirés forme la branche expiratoire du circuit patient 29.

La ligne d'alimentation 22 en gaz est raccordée fluidiquement à un port de sortie 23a du ventilateur médical de manière à récupérer et acheminer le gaz, typiquement de l'air (ou mélange $N_2/O_2$ contenant environ 21% d'$O_2$) délivré par le ventilateur médical 23, alors que la ligne de récupération 27 des gaz expirés est raccordée fluidiquement à un port d'entrée 23b du ventilateur médical 23 de manière à fournir au ventilateur médical 23 tout ou partie du débit des gaz expirés par le patient.

La ligne de récupération 27 des gaz expirés peut comprendre un ou d'autres composants 26 optionnels, comme par exemple un dispositif d'élimination du $CO_2$, i.e. un piège à $CO_2$, tel un bac à chaud ou autre, permettant d'éliminer le $CO_2$ présent dans les gaz expirés par le patient ou un filtre ou autre. En effet, un dispositif d'élimination du $CO_2$ peut être utile lorsque le gaz contient du $N_2O$ à récupérer après son exhalation par le patient. La ligne de récupération 27 des gaz expirés est utilisée, dans le cas du NO, par le ventilateur 23 pour vérifier s'il existe une fuite de gaz dans le circuit 22, 27 par exemple.

Il est aussi prévu un capteur de débit 36, par exemple du type à fil chaud ou à différentiel de pression, relié au dispositif de fourniture de gaz 1 via une ligne de mesure de débit 35 servant à mesurer le débit de gaz (Q') provenant du ventilateur 23, tel de l'air (i.e. $N_2/O_2$), au sein de la ligne d'alimentation 22, en amont du site de raccordement et de mélange $NO/N_2/$air (en 37a). Comme expliqué ci-avant, ceci permet notamment de réguler le passage du NO au travers des électrovannes 5 du dispositif 1.

Par ailleurs, on peut prévoir une ligne de prélèvement 38 de gaz reliant fluidiquement le dispositif de fourniture de gaz 1 à la ligne d'alimentation 22, à proximité de la pièce en Y 28, servant à prélever des échantillons de gaz et à vérifier leur conformité avec le mélange souhaité devant être administré au patient. La ligne de prélèvement 38 est reliée au conduit 22 en aval (en 38a) du site de liaison 37a du conduit d'injection 37, en considérant le sens de circulation du flux gazeux du ventilateur 23 vers le patient.

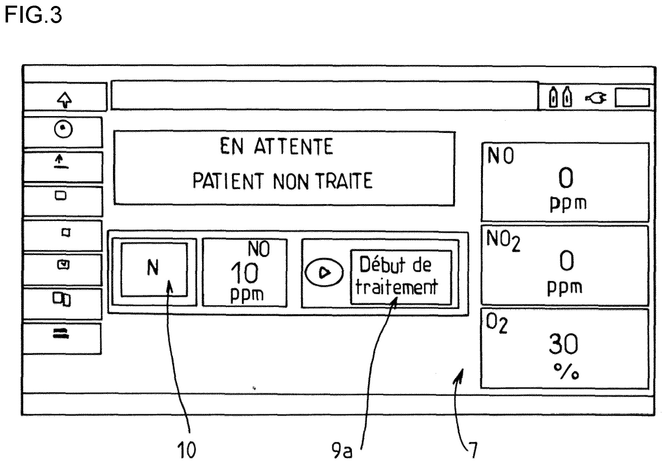

Fig. 3 schématise un mode de réalisation de l'écran de l'afficheur graphique 7 pour un dispositif 1 de fourniture de gaz selon l'invention, lequel est configuré pour afficher différentes informations, comme les concentrations en NO, en $O_2$ et en $NO_2$ dans le gaz, mais aussi, selon l'invention une touche (i.e. fenêtre) tactile de « Début de traitement » permettant à l'utilisateur, i.e. un personnel soignant, de sélectionner un premier choix 9a correspondant au début d'un traitement par administration de NO à un patient et par ailleurs, le nombre « N » (en 10) de patients ayant été traités avec ce dispositif 1.

Dans ce mode de réalisation, le ou les autres choix 9b-9d ne sont affichés qu'après sélection du premier choix 9a par l'utilisateur, i.e. après appui sur la fenêtre formant une touche tactile de l'écran tactile.

La problématique d'enregistrement du nombre de patients traités étant globale pour les centres hospitaliers publics et privés, le dispositif de l'invention peut servir à d'autres traitements que celui par monoxyde d'azote inhalé, i.e. iNO, par exemple pour les traitements à base de mélange équimolaire d'oxygène et de protoxyde d'azote, tel MEOPA, ou au moyen d'autres gaz, par exemple de l'argon.

**Revendications**

1. Dispositif de fourniture (1) de gaz thérapeutique comprenant :

- un passage interne (2) pour acheminer un flux de gaz thérapeutique entre une entrée (3) et une sortie (4) de gaz,
- des moyens à valve (5) pour contrôler le flux de gaz thérapeutique dans le passage interne (2),
- des moyens de pilotage (6) comprenant au moins un microprocesseur configurés pour contrôler au moins les moyens à valve (5),
- un afficheur graphique (7) configuré pour afficher des choix sélectionnables (9a-9d) par un utilisateur, et
- des moyens de sélection (8) comprenant une ou des touches de sélection, configurés pour opérer une sélection parmi les choix sélectionnables (9a-9d) affichés sur l'afficheur graphique (7),

**caractérisé en ce que** les moyens de pilotage (6) sont configurés pour :

i) comptabiliser un nombre total (N) de patients traités par administration du gaz thérapeutique à partir de la sélection par un utilisateur, via les moyens de sélection (8), d'un premier choix donné (9a) correspondant au début d'un traitement par administration du gaz thérapeutique à un patient considéré, ladite comptabilisation du nombre total (N) de patients traités étant opérée par incrémentation d'un compteur de patients,

ii) déterminer une durée totale de traitement pour chaque patient traité correspond à la période de temps s'écoulant entre une sélection par l'utilisateur :

- du premier choix donné (9a) correspondant au début d'un traitement par administration du gaz thérapeutique à un patient considéré et
- d'un second choix donné (9b) correspondant à la fin du traitement par administration du gaz thérapeutique audit patient considéré,

iii) déterminer, à partir du nombre total (N) de patients traités ayant été comptabilisé et des durées totales de traitement déterminées pour les patients traités:

- le nombre de traitements courts (TC) pour lesquels la durée totale de traitement est inférieure à une durée-seuil préfixée et/ou
- le nombre de traitements longs (TL) pour lesquels la durée totale de traitement est supérieure à ladite durée-seuil préfixée, et

iv) coopérer avec l'afficheur graphique (7) pour afficher (11 ; 12) le nombre total (N) de patients traités ayant été comptabilisé, le nombre de traitements courts (TC) et/ou le nombre de traitements longs (TL),

et dans lequel :

- l'afficheur graphique (7) comprend un écran tactile et la ou les touches de sélection sont une ou des touches virtuelles s'affichant sur l'écran tactile, et
- les moyens de pilotage (6) sont configurés pour commander les moyens à valve (5) et débuter une fourniture de gaz thérapeutique en réponse à une sélection par l'utilisateur du premier choix donné (9a), par appui sur une touche virtuelle s'affichant sur l'écran tactile, correspondant au début d'un traitement par administration de gaz thérapeutique au patient considéré, et pour incrémenter de +1 le compteur du nombre total (N) de patients traités, après ledit appui sur la touche virtuelle.

2. Dispositif selon la revendication 1, **caractérisé en ce que** les moyens de pilotage (6) sont configurés pour commander les moyens à valve (5) et débuter une fourniture de gaz thérapeutique en réponse à une sélection par l'utilisateur du premier choix donné (9a) correspondant au début d'un traitement par administration de gaz thérapeutique au patient considéré suivie d'une validation de ladite sélection du premier choix.

3. Dispositif selon l'une des revendications 1 ou 2, **caractérisé en ce que** les moyens de pilotage (6) comprennent au moins un microcontrôleur.

4. Dispositif selon la revendication 1, **caractérisé en ce qu'**il comprend des moyens de remise à zéro (RAZ) permettant de réinitialiser le compteur.

5. Dispositif selon la revendication 1, **caractérisé en ce que** l'afficheur graphique (7) est configuré pour afficher la ou les touches de sélection virtuelles sous forme d'une ou de fenêtres de choix.

6. Dispositif selon la revendication 1, **caractérisé en ce que** le compteur de patients est un compteur interne du microprocesseur.

**7.** Dispositif selon l'une des revendications 1 ou 6, **caractérisé en ce que** les moyens de pilotage (6) sont configurés pour incrémenter de +1, le nombre total (N) de patients traités, en réponse à une sélection par l'utilisateur du premier choix donné (9a) correspondant au début d'un traitement par administration de gaz thérapeutique à un patient considéré.

**8.** Dispositif selon la revendication 1, **caractérisé en ce que** la durée-seuil préfixée est inférieure ou égale à 5 heures, typiquement de l'ordre de 3h à 4h.

**9.** Dispositif selon la revendication 1, **caractérisé en ce que** les moyens de pilotage (6) sont en outre configurés pour :

a) interrompre temporairement la détermination de la durée totale de traitement pour un patient considéré après :

- soit sélection par l'utilisateur du second choix (9b) correspondant à la fin du traitement,
- soit sélection par l'utilisateur d'un troisième choix donné (9c) correspondant à une suspension de traitement avec interruption temporaire de l'administration de gaz thérapeutique audit patient considéré, et

b) reprendre la détermination de la durée totale de traitement pour ledit patient considéré après :

- soit re-sélection par l'utilisateur du premier choix donné (9a) correspondant au début d'un traitement par administration du gaz thérapeutique,
- soit sélection par l'utilisateur d'un quatrième choix donné (9d) correspondant à la reprise d'un traitement par administration du gaz thérapeutique chez ledit patient donné.

**10.** Dispositif selon l'une des revendications 1 et 9, **caractérisé en ce que** les moyens de pilotage (6) sont en outre configurés pour ne pas incrémenter le compteur de patients après une re-sélection par l'utilisateur du premier choix (9a) correspondant au début d'un traitement ou après une sélection du quatrième choix (9d) correspondant à une reprise de traitement chez un patient donné.

**11.** Dispositif selon la revendication 1, **caractérisé en ce que** les moyens de pilotage (6) sont configurés pour contrôler les moyens à valve (5) de manière à ajuster le débit de gaz thérapeutique circulant dans le passage interne (2).

**12.** Dispositif selon la revendication 1, **caractérisé en ce qu'**il comprend en outre des moyens de mémorisation configurés pour mémoriser le nombre total (N) de patients traités, le nombre de traitements courts (TC), le nombre de traitements longs (TL), les choix sélectionnables par l'utilisateur et la durée-seuil préfixée.

**13.** Installation d'administration de gaz (20) à un patient comprenant :

- au moins une source de gaz thérapeutique (21),
- un dispositif de fourniture (1) de gaz thérapeutique selon l'une des revendications précédentes, alimenté en gaz thérapeutique par ladite au moins une source de gaz thérapeutique (21), et
- une ligne d'alimentation en gaz (22) alimentée en gaz thérapeutique par le dispositif de fourniture (1) de gaz thérapeutique.

**14.** Installation selon la revendication 13, **caractérisé en ce qu'**elle comporte en outre un ventilateur médical (23) en communication fluidique avec la ligne d'alimentation en gaz (22).

**15.** Installation selon la revendication 13, **caractérisé en ce qu'**au moins une source de gaz thérapeutique (21) contient un mélange $NO/N_2$ ou $O_2/N_2O$, en particulier un ou plusieurs récipients de gaz.

**Patentansprüche**

**1.** Zuführvorrichtung (1) für therapeutisches Gas, umfassend:

- einen internen Durchlass (2), um einen Strom von therapeutischem Gas zwischen einem Gaseinlass (3) und einem Gasauslass (4) weiterzuleiten,

- Ventilmittel (5), um den Strom von therapeutischem Gas in dem internen Durchlass (2) zu steuern,
- Ansteuerungsmittel (6), die mindestens einen Mikroprozessor umfassen und dazu ausgestaltet sind, mindestens die Ventilmittel (5) zu steuern,
- ein Grafikdisplay (7), das dazu ausgestaltet ist, von einem Benutzer auswählbare Wahlmöglichkeiten (9a-9d) anzuzeigen, und
- Auswahlmittel (8), die eine oder mehrere Auswahltasten umfassen und dazu ausgestaltet sind, eine Auswahl unter den auf dem Grafikdisplay (7) angezeigten auswählbaren Wahlmöglichkeiten (9a-9d) vorzunehmen,

**dadurch gekennzeichnet, dass** die Ansteuerungsmittel (6) dazu ausgestaltet sind:

i) eine Gesamtzahl (N) von Patienten, die durch Verabreichung des therapeutischen Gases behandelt wurden, ausgehend von dem Auswählen durch einen Benutzer, über die Auswahlmittel (8), einer gegebenen ersten Wahlmöglichkeit (9a), die dem Beginn einer Behandlung durch Verabreichung des therapeutischen Gases bei einem betrachteten Patienten entspricht, zu erfassen, wobei die Erfassung der Gesamtzahl (N) behandelter Patienten durch Inkrementieren eines Patientenzählers vorgenommen wird,
ii) eine Behandlungsgesamtdauer für jeden behandelten Patienten zu bestimmen, die dem Zeitraum entspricht, der vergeht zwischen einem Auswählen durch den Benutzer:

- der gegebenen ersten Wahlmöglichkeit (9a), die dem Beginn einer Behandlung durch Verabreichung des therapeutischen Gases bei einem betrachteten Patienten entspricht, und
- einer gegebenen zweiten Wahlmöglichkeit (9b), die dem Ende der Behandlung durch Verabreichung des therapeutischen Gases bei dem betrachteten Patienten entspricht,

iii) ausgehend von der Gesamtzahl (N) behandelter Patienten, die erfasst worden ist, und von den Behandlungsgesamtdauern, die für die behandelten Patienten bestimmt wurden, zu bestimmen:

- die Anzahl kurzer Behandlungen (TC), bei denen die Behandlungsgesamtdauer unter einer zuvor festgelegten Schwellenwertdauer liegt, und/oder
- die Anzahl langer Behandlungen (TL), bei denen die Behandlungsgesamtdauer über der zuvor festgelegten Schwellenwertdauer liegt, und

iv) mit dem Grafikdisplay (7) zusammenzuwirken, um die Gesamtzahl (N) behandelter Patienten, die erfasst worden ist, die Anzahl kurzer Behandlungen (TC) und/oder die Anzahl langer Behandlungen (TL) anzuzeigen (11, 12), und bei der:

- das Grafikdisplay (7) einen Touchscreen umfasst und die Auswahltaste oder die Auswahltasten ein oder mehrere virtuelle Tasten sind, die auf dem Touchscreen angezeigt werden, und
- die Ansteuerungsmittel (6) dazu ausgestaltet sind, die Ventilmittel (5) zu betätigen und eine Zuführung von therapeutischem Gas als Antwort auf ein Auswählen durch den Benutzer der gegebenen ersten Wahlmöglichkeit (9a) durch Drücken auf eine auf dem Touchscreen angezeigte virtuelle Taste, die dem Beginn einer Behandlung durch Verabreichung von therapeutischem Gas bei dem betrachteten Patienten entspricht, zu beginnen und den Zähler für die Gesamtzahl (N) behandelter Patienten nach dem Drücken auf die virtuelle Taste um +1 zu inkrementieren.

2. Vorrichtung nach Anspruch 1, **dadurch gekennzeichnet, dass** die Ansteuerungsmittel (6) dazu ausgestaltet sind, die Ventilmittel (5) zu betätigen und eine Zuführung von therapeutischem Gas als Antwort auf ein Auswählen durch den Benutzer der gegebenen ersten Wahlmöglichkeit (9a), die dem Beginn einer Behandlung durch Verabreichung von therapeutischem Gas bei dem betrachteten Patienten entspricht, mit anschließender Bestätigung der Auswahl der ersten Wahlmöglichkeit zu beginnen.

3. Vorrichtung nach einem der Ansprüche 1 oder 2, **dadurch gekennzeichnet, dass** die Ansteuerungsmittel (6) mindestens einen Mikrocontroller umfassen.

4. Vorrichtung nach Anspruch 1, **dadurch gekennzeichnet, dass** sie Mittel zur Nullrückstellung (RAZ) umfasst, die es ermöglichen, den Zähler zurückzusetzen.

5. Vorrichtung nach Anspruch 1, **dadurch gekennzeichnet, dass** das Grafikdisplay (7) dazu ausgestaltet ist, die virtuelle Auswahltaste oder die virtuellen Auswahltasten in Form eines oder mehrerer Fenster mit Wahlmöglichkeiten

14

anzuzeigen.

6. Vorrichtung nach Anspruch 1, **dadurch gekennzeichnet, dass** der Patientenzähler ein interner Zähler des Mikroprozessors ist.

7. Vorrichtung nach einem der Ansprüche 1 oder 6, **dadurch gekennzeichnet, dass** die Ansteuerungsmittel (6) dazu ausgestaltet sind, die Gesamtzahl (N) behandelter Patienten als Antwort auf ein Auswählen durch den Benutzer der gegebenen ersten Wahlmöglichkeit (9a), die dem Beginn einer Behandlung durch Verabreichung von therapeutischem Gas bei einem betrachteten Patienten entspricht, um +1 zu inkrementieren.

8. Vorrichtung nach Anspruch 1, **dadurch gekennzeichnet, dass** die zuvor festgelegte Schwellenwertdauer kleiner oder gleich 5 Stunden ist, typischerweise in der Größenordnung von 3 h bis 4 h liegt.

9. Vorrichtung nach Anspruch 1, **dadurch gekennzeichnet, dass** die Ansteuerungsmittel (6) ferner dazu ausgestaltet sind:

a) das Bestimmen der Behandlungsgesamtdauer bei einem betrachteten Patienten vorübergehend zu unterbrechen nach:

- entweder dem Auswählen durch den Benutzer der zweiten Wahlmöglichkeit (9b), die dem Ende der Behandlung entspricht,
- oder dem Auswählen durch den Benutzer einer gegebenen dritten Wahlmöglichkeit (9c), die einer Behandlungsaussetzung mit vorübergehender Unterbrechung der Verabreichung von therapeutischem Gas bei dem betrachteten Patienten entspricht, und

b) das Bestimmen der Behandlungsgesamtdauer für den betrachteten Patienten wiederaufzunehmen nach:

- entweder dem erneuten Auswählen durch den Benutzer der gegebenen ersten Wahlmöglichkeit (9a), die dem Beginn einer Behandlung durch Verabreichung des therapeutischen Gases entspricht,
- oder dem Auswählen durch den Benutzer einer gegebenen vierten Wahlmöglichkeit (9d), die der Wiederaufnahme einer Behandlung durch Verabreichung des therapeutischen Gases bei dem gegebenen Patienten entspricht.

10. Vorrichtung nach einem der Ansprüche 1 und 9, **dadurch gekennzeichnet, dass** die Ansteuerungsmittel (6) ferner dazu ausgestaltet sind, den Patientenzähler nach einem erneuten Auswählen durch den Benutzer der ersten Wahlmöglichkeit (9a), die dem Beginn einer Behandlung entspricht, oder nach einem Auswählen der vierten Wahlmöglichkeit (9d), die einer Behandlungswiederaufnahme bei einem gegebenen Patienten entspricht, nicht zu inkrementieren.

11. Vorrichtung nach Anspruch 1, **dadurch gekennzeichnet, dass** die Ansteuerungsmittel (6) dazu ausgestaltet sind, die Ventilmittel (5) so zu steuern, dass der in dem internen Durchlass (2) zirkulierende Volumenstrom von therapeutischem Gas angepasst wird.

12. Vorrichtung nach Anspruch 1, **dadurch gekennzeichnet, dass** sie ferner Speichermittel umfasst, die dazu ausgestaltet sind, die Gesamtzahl (N) behandelter Patienten, die Anzahl kurzer Behandlungen (TC), die Anzahl langer Behandlungen (TL), die vom Benutzer auswählbaren Wahlmöglichkeiten und die zuvor festgelegte Schwellenwertdauer zu speichern.

13. Anlage zur Verabreichung von Gas (20) bei einem Patienten, umfassend:

- mindestens eine Quelle für therapeutisches Gas (21),
- eine Zuführvorrichtung (1) für therapeutisches Gas nach einem der vorhergehenden Ansprüche, die von der mindestens einen Quelle für therapeutisches Gas (21) mit therapeutischem Gas versorgt wird, und
- eine Gasversorgungsleitung (22), die von der Zuführvorrichtung (1) für therapeutisches Gas mit therapeutischem Gas versorgt wird.

14. Anlage nach Anspruch 13, **dadurch gekennzeichnet, dass** sie ferner ein medizinisches Beatmungsgerät (23) beinhaltet, das mit der Gasversorgungsleitung (22) in Fluidverbindung steht.

**15.** Anlage nach Anspruch 13, **dadurch gekennzeichnet, dass** mindestens eine Quelle für therapeutisches Gas (21) ein NO/N$_2$- oder O$_2$/N$_2$O-Gemisch enthält, insbesondere einen oder mehrere Gasbehälter.

**Claims**

**1.** Device (1) for supplying therapeutic gas, comprising:

- an internal passage (2) for conveying a flow of therapeutic gas between a gas inlet (3) and a gas outlet (4),
- valve means (5) for controlling the flow of therapeutic gas in the internal passage (2),
- control means (6) comprising at least a microprocessor and configured to control at least the valve means (5),
- a graphic display (7) configured to display choices (9a-9d) that can be selected by a user, and
- selection means (8) comprising one or more selection keys configured to make a selection from among the selectable choices (9a-9d) displayed on the graphic display (7),

**characterized in that** the control means (6) are configured to:

i. count a total number (N) of patients treated by administration of the therapeutic gas from the selection, by a user, via the selection means (8), of a first given choice (9a) corresponding to the start of a treatment by administering the therapeutic gas to a patient concerned, said counting of the total number (N) of patients treated being performed by incrementing a patients counter,

ii. determine a total duration of treatment for each patient treated which corresponds to the period of time elapsed between the user selecting:

- the first given choice (9a) corresponding to the start of a treatment by administering the therapeutic gas to a patient concerned, and
- a second given choice (9b) corresponding to the end of the treatment by administering the therapeutic gas to said patient concerned,

iii. determine, from the total number (N) of patients treated, which has been counted up, and from the total treatment durations determined for the treated patients:

- the number of short treatments (TC) for which the total treatment duration is below a preset threshold duration, and/or
- the number of long treatments (TL) for which the total treatment duration is above said preset threshold duration, and

iv. cooperate with the graphic display (7) to display (11; 12) the total number (N) of patients treated, which has been counted up, the number of short treatments (TC) and/or the number of long treatments (TL),

and wherein:

- the graphic display (7) comprises a touchscreen and the selection key or keys are virtual keys displayed on the touchscreen, and
- the control means (6) are configured to control the valve means (5) and to start to supply therapeutic gas in response to a selection, by the user, of the first given choice (9a), by pressing a virtual key displayed on the touchscreen and corresponding to the start of a treatment by administering therapeutic gas to the patient concerned, and to increment the total number (N) of patients treated by +1 once the virtual key has been pressed.

**2.** Device according to Claim 1, **characterized in that** the control means (6) are configured to control the valve means (5) and to start to supply therapeutic gas in response to a selection, by the user, of the first given choice (9a) corresponding to the start of a treatment by administering therapeutic gas to the patient concerned, followed by a validation of said selection of the first choice.

**3.** Device according to either of Claims 1 and 2, **characterized in that** the control means (6) comprise at least one microcontroller.

**4.** Device according to Claim 1, **characterized in that** it comprises zero-reset (RAZ) means enabling the counter to be reset.

5. Device according to Claim 1, **characterized in that** the graphic display (7) is configured to display the virtual selection key or keys in the form of one or more selection windows.

6. Device according to Claim 1, **characterized in that** the patients counter is a counter internal to a microprocessor.

7. Device according to either of Claims 1 and 6, **characterized in that** the control means (6) are configured to increment the total number (N) of patients treated by +1 in response to a selection, by the user, of the first given choice (9a) corresponding to the start of a treatment by administering therapeutic gas to a patient concerned.

8. Device according to Claim 1, **characterized in that** the preset threshold duration is less than or equal to 5 hours, typically of the order of 3h to 4h.

9. Device according to Claim 1, **characterized in that** the control means (6) are also configured to:

   a) temporarily interrupt the determining of the total duration of treatment for a patient concerned after:

   - either the user selects the second choice (9b) corresponding to the end of the treatment,
   - or the user selects a third given choice (9c) corresponding to a suspension of treatment with temporary interruption of the administration of therapeutic gas to said patient concerned, and

   b) resume determining the total duration of treatment for said patient concerned after:

   - either the user reselects the first given choice (9a) corresponding to the start of a treatment by administering the therapeutic gas,
   - or the user selects a fourth given choice (9d) corresponding to resumption of a treatment by administering the therapeutic gas to said given patient.

10. Device according to either of Claims 1 and 9, **characterized in that** the control means (6) are also configured not to increment the patients counter after the user reselects the first choice (9a) corresponding to the start of a treatment or selects the fourth choice (9d) corresponding to the resumption of treatment for a given patient.

11. Device according to Claim 1, **characterized in that** the control means (6) are configured to control the valve means (5) so as to adjust the flowrate of therapeutic gas circulating in the internal passage (2).

12. Device according to Claim 1, **characterized in that** it further comprises memory storage means configured to store the total number (N) of patients treated, the number of short treatments (TC), the number of long treatments (TL), the choices selectable by the user and the preset threshold duration.

13. Installation (20) for administering gas to a patient, comprising:

   - at least one source of therapeutic gas (21),
   - a therapeutic-gas supply device (1) according to one of the preceding claims, supplied with therapeutic gas by said at least one source of therapeutic gas (21), and
   - a gas supply line (22) supplied with therapeutic gas by the therapeutic-gas supply device (1).

14. Installation according to Claim 13, **characterized in that** it further comprises a medical ventilator (23) in fluidic communication with the gas supply line (22).

15. Installation according to Claim 13, **characterized in that** at least one source of therapeutic gas (21) contains an $NO/N_2$ or $O_2/N_2O$ mixture, particularly one or several gas containers.

FIG.1

FIG.2

## FIG.3

EN ATTENTE
PATIENT NON TRAITE

N

NO
10
ppm

▶ Début de
traitement

NO
0
ppm

NO$_2$
0
ppm

O$_2$
30
%

10          9a          7

## RÉFÉRENCES CITÉES DANS LA DESCRIPTION

**Documents brevets cités dans la description**

- EP 560928 A
- EP 1516639 A
- WO 2012094008 A
- US 2015320951 A
- US 2015273175 A
- JP H11192303 A
- WO 0240914 A
- US 2003116159 A